# EUROPEAN PATENT SPECIFICATION

(11) **EP 0 290 165 B1**
(45) Date of publication and mention of the grant of the patent: **16.03.1994**
(21) Application number: 88303568.5
(22) Date of filing: 20.04.1988
(51) Int. Cl.: A61B 6/06

(54) **Radiography apparatus**
Gerät für Röntgenaufnahme
Appareil de radiographie

(30) Priority: 07.05.1987 US 46860
(43) Date of publication of application: 09.11.1988
(73) Proprietor: PICKER INTERNATIONAL, INC., Highland Heights Ohio 44143 (US)
(72) Inventor: Mattson, Rodney A., Mentor Ohio 44060 (US); Levar, Robert E., Willoughby Ohio 44094 (US)
(74) Representative: Pope, Michael Bertram Wingate

(56) References cited:
- CA-A- 1 147 069
- DE-C- 913 678
- FR-A- 2 398 487
- US-A- 3 631 249
- US-A- 4 195 229

## Description

This invention relates to the field of radiography. It finds particular application in conjunction with x-ray tubes for computed tomographic scanners and will be described with particular reference thereto. However, it will be appreciated that the invention may find further application in other areas of radiography, such as medical diagnostic digital x-ray, conventional x-ray, radiation therapy, and the like.

In computed tomography (CT), a slice of a patient to be examined is disposed in a scan circle of a scanner. A fan shaped x-ray beam is projected from an x-ray tube through a shutter, a collimator, the scan circle, and the patient slice to an array of radiation detectors. By rotating the radiation source, shutter, and collimator relative to the patient, radiation is projected through the imaged slice to the detectors from a multiplicity of directions. From radiation intensity data sampled at the detectors, data indicative of the path the sampled radiation traveled to reach each sampled detector, and other data, an image of the examined slice of the patient is reconstructed.

One of the problems encountered in CT scanners is the deleterious effect of off-focal radiation. In the x-ray tube, an electron beam strikes a focal spot point or line on an anode. X-rays are generated at this focal spot and travel along diverging linear paths in an x-ray fan beam of dimension controlled by the collimator. If all the radiation were emitted from the focal spot, then the path traveled by each x-ray beam from the x-ray tube to the detector for each detector sampling could be accurately determined. However, x-rays are emitted from regions of the anode other than the focal spot. In CT scanning x-ray tubes, 3% to 8% of the detected radiation is commonly off-focal radiation, i.e. radiation not originating at the focal spot. The spread in the origin of the radiation from off-focal radiation causes small objects and sharp edges to lose proper definition and become blurred. The lack of definition gives rise to non-linear artifacts in the reconstructed image.

The CT scanner collimators are commonly disposed adjacent the scan circle, i.e. displaced from the x-ray tube anode. Although this placement assures accurate beam dimensions in the scan circle, off-focal radiation originating on portions of the anode well displaced from the focal spot are permitted to pass through the collimator to the detectors. The greater the distance between the anode and the collimator, the greater the parallax and off-focal radiation may originate more distantly from the focal spot and still pass through the collimator to the detectors, i.e. the more blurred the focal spot becomes.

The off-focal radiation has a particularly deleterious effect on the bone correction applied to brain scans. A calcium correction is commonly made to minimize the effects attributable to radiation spectrum changes due to absorption by the bone tissue. The calcium correction deconvolves the effect of the broad spectrum radiation source's projection across the bone/brain interface on the soft tissue. The variety of sizes, shapes, and density of skulls which may be examined in common clinical practice renders impractical a universal correction for the effects of off-focal radiation and beam hardening.

US-A 4195229 discloses an x-ray photographing apparatus including an x-ray tube for generating an x-ray pulse beam, a shutter for shutting off the x-ray pulse beam which is emitted from the x-ray tube, and a shutter controller for permitting the x-ray pulse beam shut off by the shutter to be passed therethrough when the intensity of the x-ray pulse beam from the x-ray tube reaches a substantially constant level.

The present invention provides a radiography scanner for reducing off-focal radiation for smaller scans without affecting reduction for larger scans.

In accordance with the present invention there is provided a radiographic scanner comprising: a source of penetrating radiation for projecting a generally fan shaped beam of radiation; a shutter means disposed between said radiation source and a patient receiving region for selectively gating the radiation beam therepast to irradiate a selected region of a patient; a beam collimating means disposed between said radiation source and the patient receiving region for accurately defining the dimensions of the radiation beam; radiation detecting means for receiving radiation from said radiation source which has transversed the patient; and a radiation absorbing member which is positioned between said radiation source and said shutter means characterised by: an off-focal control means including said radiation absorbed means which has at least two differently dimensioned radiation passing portions; and moveable mounting means for moveably mounting the radiation absorbing member adjacent the radiation source such that either one of the radiation passing portions is selectively moveable into alignment with the radiation beam and wherein the moveable mounting means mounts the radiation absorbing member for rotation about an axis which is generally parallel to a central axis of the radiation beam and the radiation absorbing member is a plate and the first and second radiation passing portions are slots which cross at the axis of rotation.

One advantage of the scanner embodying the present invention is that it reduces off-focal radiation, particularly during small scans, e.g. head scans.

Another advantage of the scanner embodying the present invention is that it is disposed within the confines of a conventional x-ray tube assembly. This simplifies and facilitates installation, particularly the retrofitting of scanners already in place.

Yet another advantage of the scanner embodying the present invention is that it selectively adjusts the fan angle or other dimensions of a beam of x-rays to accommodate the examination of regions of different sizes.

One radiography apparatus and method in accordance with the invention will now be described, by way of example, with reference to the accompanying drawings in which:-
Figure 1 is a diagrammatic illustration of a computed tomography scanner in accordance with the present invention;
Figure 2 is a side view in partial section of an x-ray tube assembly of the scanner including an adjustable off-focal radiation control means, a shutter, and a collimator assembly;
Figure 3 is an enlarged view of an x-ray exit port of the x-ray tube of Figure 2 including the adjustable off-focal radiation control means;
Figure 4 is a sectional view through section 4-4 of Figure 2; and
Figure 5 is a bottom view of the collimator assembly of Figure 2.

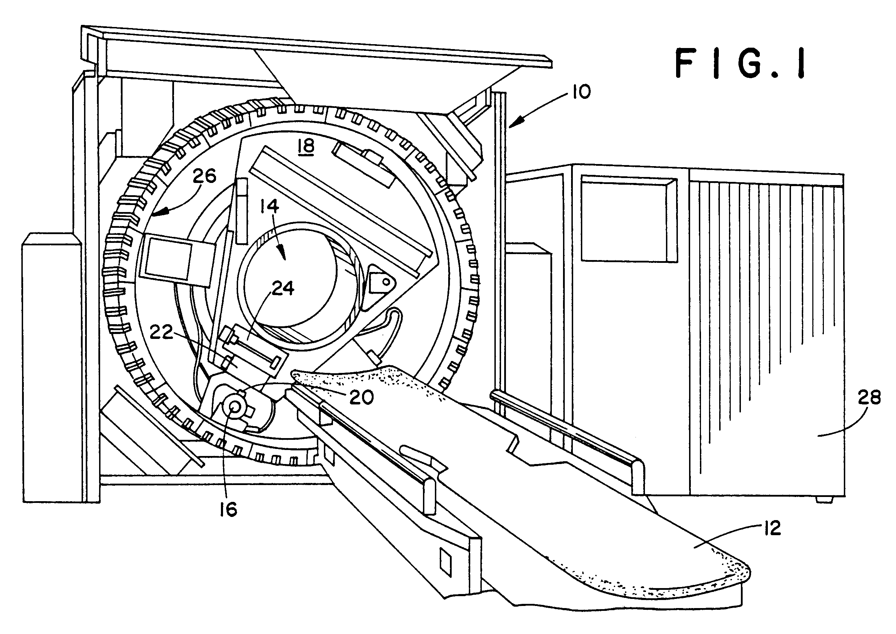

With reference to Figure 1, a computed tomography scanner 10 selectively images cross sectional slices of a region of a patient supported on a patient couch 12 within a scan circle or patient aperture 14. An x-ray tube 16 for emitting a fan shaped beam of radiation toward and spanning the scan circle 14 is mounted to a rotatable gantry 18. An off-focal radiation collimator or control means 20 is mounted to a radiation port of the x-ray tube to attenuate off-focal radiation from reaching the scan circle. A shutter 22 selectively gates the x-ray beam to permit and block it from reaching the scan circle. A primary collimator 24 selectively adjusts the dimensions of the x-ray beam, particularly its width to select the thickness of the slice which is imaged. An array of radiation detectors 26 is disposed opposite the scan circle from the x-ray tube to receive and convert radiation which has traversed the scan circle into data indicative of x-ray intensity. An image reconstruction means 28 such as the high speed data processing computer reconstructs one or more image representations from the intensity data.

In operation, a patient disposed on the patient supporting couch 12 is moved into the patient receiving region until the region of interest is appropriately positioned in the scan circle. The shutter 22 allows the fan shaped beam of radiation from the x-ray tube to traverse the scan circle 14 as an electrical motor (not shown) commences rotating the gantry 18. As the gantry rotates, the radiation detectors 26 each receive radiation along a series of paths. The computer 28 reconstructs a two dimensional image representation of the examined patient slice from the sampled x-ray detector radiation intensity data, data indicative of the position of the x-ray source, and the like.

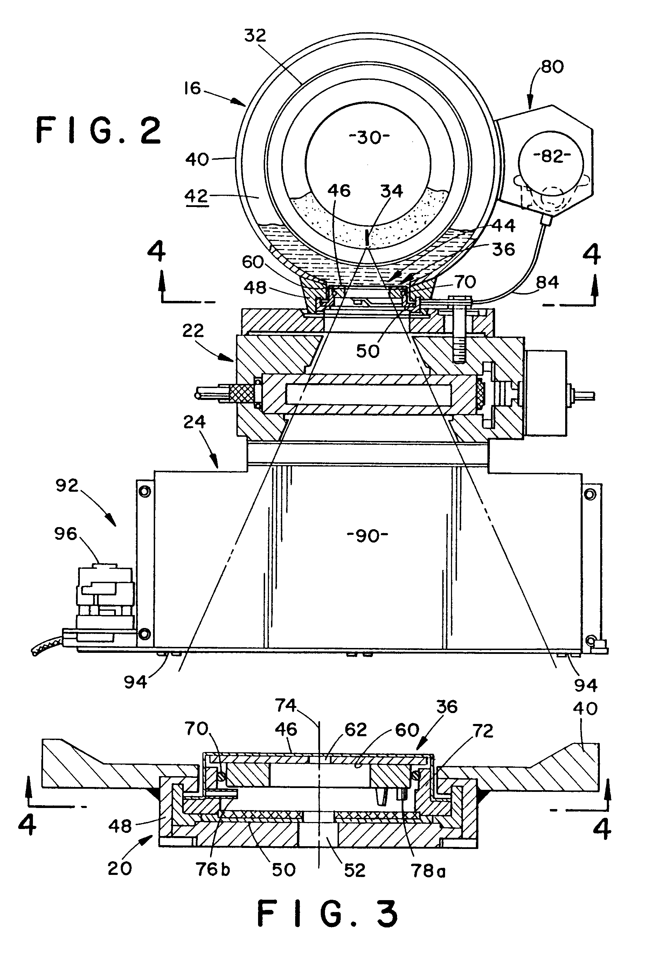

With reference to Figure 2, the x-ray tube 16 includes an anode 30 which is rotatably mounted within an evacuated glass envelope 32. A high potential difference accelerates a beam of electrons from a cathode (not shown) to the rotating anode 30. The cathode and other tube structure act to focus the beam of electrons to a rectangular or linear region (or other preselected shape) to form a focal spot 34. The anode is typically made of tungsten or similar metal with a high melting point. The deceleration of electrons as they strike the anode produces poly-energetic x-radiation. The radiation produced is multi-directional but generally propagates a beam toward an x-ray port 36 due to the inclined face of the anode. Scattered electrons and radiation of a commensurate energy strike the anode at regions displaced from the focal spot causing the off-focal radiation to be emitted.

A metal housing 40 surrounds the glass envelope 32 to define an oil receiving reservoir 42 therebetween. Oil from within the reservoir is commonly circulated to a cooling system (not shown) to control the temperature of the x-ray tube. The x-ray port 36 is defined by an aperture 44 in the housing which is sealed by a fluid impermeable, x-ray transmissive window 46, e.g. an aluminum sheet. The housing includes a collar 48 which surrounds the x-ray port 36 for greater structural strength.

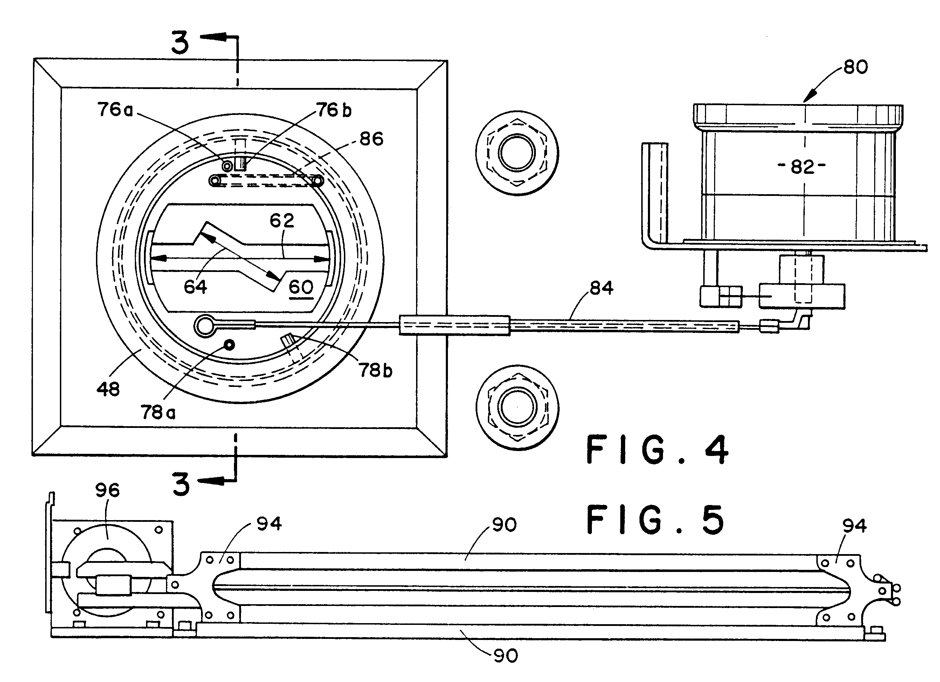

With continuing reference to FIGURE 2 and particular reference to FIGURES 3 and 4, the adjustable, off-focal collimator or control means 20 is mounted within the collar 48 which surrounds the x-ray port 36. The aluminum window 46 is mounted at an inner or near end of the collar and a first stationary, radiation or blocking plate or member 50 is mounted stationarily to a distal end of the collar. The first, stationary plate 50 is constructed of a dense x-ray absorbing material or materials. For example, the plate may include one or more layers of lead supported on a structurally stronger material which may also have high energy absorbing properties. The first stationary plate 50 includes an x-ray passing region 52, such as an elongated slot. The length and width of the x-ray passing region limit the span and thickness of the x-ray beam and to a limited extent block off-focal radiation from reaching the radiation sensors 26.

A second moveable radiation attentuation or blocking member or plate 60 is moveably mounted in the collar. The second, moveable plate is again constructed of a dense x-ray absorbing material, such as tungsten, tantalum, or reinforced lead alloys. The second, moveable plate defines at least a first or whole body scan radiation passing portion 62 and a second or head scan radiation passing portion 64. The second, moveable plate is mounted as close to the focal spot 34 as the aluminum window 46 permits. The closer the second, moveable plate is mounted to the focal spot, the more completely it blocks off-focal radiation. In the preferred embodiment, the first radiation passing portion is a slot or aperture portion which permits the beam to have a fan angle of about 41°, sufficient to span the scan circle 14 for whole body imaging. The second radiation passing portion is a shorter slot or aperture portion which limits the fan to a span of about 25 1/2° for head scans. The shorter slot blocks the radiation which is unused in reconstructing a head image from reaching the patient receiving area to reduce scattered radiation and the potential x-ray dose which the patient might receive.

The moveable plate 60 is mounted by a bearing 70 or other moveable mounting means to a stationary supporting structure 72 which is affixed to the collar 48. The bearing enables the moveable plate to rotate about a central beam axis 74 at which the first and second energy passing portions cross. A first stop pin arrangement 76a, 76b limits rotational movement of the plate in one direction to a first position in which the first radiation passing portion 62 is aligned with the stationary radiation passing region 52. A second pin arrangement 78a, 78b, limits rotation of the moveable plate in the other direction to a second position in which the second radiation passing portion 64 is aligned with the stationary radiation passing region 52.

A plate moving or rotating means 80 includes an electrical motor 82 which selectively retracts a cable 84. Retraction of the cable is limited by the second set of interacting stop pins 78a, 78b. A spring 86 returns the moveable plate to the first position. In this manner, the spring provides a return to the first position whenever power is terminated.

With reference again to FIGURE 2 and further reference to FIGURE 5, the fan beam of radiation from the off-focal collimator 20 is gated by the shutter 22 to the collimator 24. The primary collimator assembly 24 includes a pair of radiation absorbing vanes 90 which lie in a common plane parallel to the path of the x-ray beam. Vane mounting means 92 movably support the vanes such that the vanes are moveable toward and away from each other. The vane mounting structure includes end plates 94 of a radiation absorbing material which define the overall width of the x-ray fan beam. As is conventional, the spacing of the end plates 94 is selected such that the fan beam of radiation spans the scan circle. A collimator adjustment motor 96 provides the motive power to adjust the width defined between the collimator vanes 90.

The invention has been described with reference to the preferred embodiments. Obviously, modifications and alterations will occur to others upon reading and understanding the preceding detailed description. It is intended that the invention be construed as including all such alterations and modifications insofar as they come within the scope of the appended claims or the equivalents thereof.

## Claims

1. A radiographic scanner comprising: a source (16) of penetrating radiation for projecting a generally fan shaped beam of radiation; a shutter means (22) disposed between said radiation source (16) and a patient receiving region (14) for selectively gating the radiation beam therepast to irradiate a selected region of a patient; a beam collimating means (24) disposed between said radiation source and the patient receiving region (14) for accurately defining the dimensions of the radiation beam; radiation detecting means (26) for receiving radiation from said radiation source (16) which has transversed the patient; and a radiation absorbing member (60) which is positioned between said radiation source (16) and said shutter means (22) characterised by: an off-focal control means (20) including said radiation absorbing member (60) which has at least two differently dimensioned radiation passing portions (62, 64); and moveable mounting means (70) for moveably mounting the radiation absorbing member (60) adjacent the radiation source (16) such that either one of the radiation passing portions (62, 64) is selectively moveable into alignment with the radiation beam and wherein the moveable mounting means (70) mounts the radiation absorbing member (60) for rotation about an axis which is generally parallel to a central axis (74) of the radiation beam and the radiation absorbing member (60) is a plate and the first and second radiation passing portions (62, 64) are slots which cross at the axis of rotation.

2. A scanner according to Claim 1 further including stop means (76, 78) for limiting rotation of the radiation absorbing member (60) between a first position and a second position.

3. A scanner according to claim 1 or 2 further including a cable (84) which interconnects the radiation absorbing member (60) with a source of motive power (82) for selectively moving the radiation absorbing member (60).

4. A scanner according to any one of claims 1 to 3, wherein said off-focal control means (20) further includes a stationary radiation absorbing member (50) mounted adjacent the moveable radiation absorbing member (60) and having a radiation passing slot (52) therein for passing the radiation therethrough, one radiation passing portion (62) of the moveable radiation absorbing member (60) being aligned with stationary slot (52) in one position and another radiation passing portion (64) being aligned with the stationary slot (52) in another position.

## Patentansprüche

1. Röntgenstrahlen-Scanner mit einer durchdringende Strahlen abgebenden Quelle (16), die ein im wesentlichen fächerförmiges Strahlenbündel aussendet, einem Verschluß (22), der zwischen der Strahlenquelle (16) und einem Patientenaufnahmebereich (14) für das selektive Hindurchlassen des Strahlenbündels angeordnet ist, um einen bestimmten Körperbereich eines Patienten zu bestrahlen, einem Strahlkollimator (24), angebracht zwischen der Strahlenquelle und dem Patientenaufnahmebereich (14) zur genauen Definition der Strahlenbündelabmessungen, einem Strahlendetektor (26) für den Empfang der durch den Patienten hindurchgetretenen Strahlung der Strahlenquelle (16) und einem Strahlenabsorberelement (60), das zwischen der Strahlenquelle (16) und dem Verschluß (22) positioniert ist,
**dadurch gekennzeichnet,**
daß eine Brennpunktabweich-Regeleinrichtung (20) aus dem Strahlenabsorberelement (60), das zumindest zwei unterschiedlich dimensionierte Strahlendurchlaßteile (62, 64) aufweist, und einem beweglichen Befestigungselement (70) besteht, das das Strahlenabsorberelement (60) nahe der Strahlenquelle (16) derart bewegbar anbringt, daß einer der Strahlendurchlaßteile (62, 64) wahlweise bis zum Fluchten mit dem Strahlenbündel bewegbar ist und daß das bewegliche Befestigungselement (70) das Strahlenabsorberelement (60) für die Rotation um eine Achse an dieser befestigt, die im wesentlichen parallel zu einer Zentralachse (74) des Strahlenbündels verläuft und das Strahlenabsorberelement (60) eine Platte und das erste und zweite Strahlendurchlaßteil (62, 64) Schlitze sind, die die Drehachse kreuzen.

2. Röntgenstrahlen-Scanner nach Anspruch 1,
**dadurch gekennzeichnet,**
daß Halteelemente (76, 78) zum Begrenzen der Drehung des Strahlenabsorberelements (60) zwischen einer ersten und zweiten Position vorhanden sind.

3. Röntgenstrahlen-Scanner nach Anspruch 1 oder 2,
**dadurch gekennzeichnet,**
daß ein Kabel (84) das Strahlenabsorberelement (60) mit einer Antriebsquelle (82) für das selektive Bewegen des Strahlenabsorberelements (60) verbindet.

4. Röntgenstrahlen-Scanner nach jedem der Ansprüche 1 bis 3,
**dadurch gekennzeichnet,**
daß die Brennpunktabweich-Regeleinrichtung (20) des weiteren ein stationäres Strahlenabsorberelement (50) aufweist, das nahe dem beweglichen Strahlenabsorberelement (60) angeordnet ist und einen Strahldurchlaßschlitz (52) für den Durchtritt der Strahlung besitzt, wobei der eine Strahlendurchlaßteil (62) mit dem stationären Schlitz (52) in einer Position und der andere Strahlendurchlaßteil (64) mit dem stationären Schlitz in einer anderen Position fluchtet.

## Revendications

1. Scanographe comprenant une source (16) d'un rayonnement pénétrant et destiné à projeter un faisceau du rayonnement ayant une forme générale d'éventail, un dispositif obturateur (22) disposé entre la source du rayonnement (16) et une région (14) de réception d'un patient pour la transmission sélective du faisceau de rayonnement afin qu'il puisse irradier une région choisie d'un patient, un dispositif (24) de collimation de faisceau disposé entre la source du rayonnement et la région (14) de réception de patient afin qu'il délimite avec précision les dimensions du faisceau du rayonnement, un dispositif (26) de détection de rayonnement destiné à recevoir le rayonnement de la source (16) qui a traversé le patient, et un organe (60) d'absorption de rayonnement qui est placé entre la source du rayonnement (16) et le dispositif obturateur (22), caractérisé par un dispositif (20) de réglage de défocalisation comprenant un organe (60) d'absorption du rayonnement qui a au moins deux parties (62, 64) de passage du rayonnement dont les dimensions sont différentes, et un dispositif mobile de montage (70) destiné au montage mobile de l'organe (60) d'absorption du rayonnement près de la source (16) du rayonnement afin que l'une des parties (62, 64) de passage de rayonnement soit mobile sélectivement afin qu'elle s'aligne sur le faisceau du rayonnement, et dans lequel le dispositif (70) de montage mobile porte l'organe (60) d'absorption du rayonnement afin qu'il tourne autour d'un axe qui est parallèle de façon générale à un axe central (74) du faisceau du rayonnement, et l'organe (60) d'absorption du rayonnement est une plaque, et la première et la seconde partie (62, 64) de passage du rayonnement sont des fentes qui se recoupent sur l'axe de rotation.

2. Scanographe selon la revendication 1, comprenant en outre un dispositif d'arrêt (76, 78) destiné à limiter la rotation de l'organe (60) d'absorption du rayonnement entre une première et une seconde position.

3. Scanographe selon la revendication 1 ou 2, comprenant en outre un câble (84) assurant l'interconnexion de l'organe (60) d'absorption du rayonnement à une source d'énergie motrice (82) afin qu'il déplace sélectivement l'organe (60) d'absorption du rayonnement.

4. Scanographe selon l'une quelconque des revendications 1 à 3, dans lequel le dispositif (20) de commande de défocalisation comporte en outre un organe fixe (50) d'absorption du rayonnement monté près de l'organe mobile (60) d'absorption du rayonnement et ayant une fente (52) de passage du rayonnement destinée à transmettre le rayonnement, une première partie (62) de passage du rayonnement de l'organe mobile (60) d'absorption du rayonnement étant alignée sur la fente fixe (52) dans une première position et l'autre partie (64) de passage du rayonnement étant alignée sur la fente fixe (52) dans l'autre position.
